# EUROPEAN PATENT SPECIFICATION

(11) **EP 2 688 649 B1**
(45) Date of publication and mention of the grant of the patent: **10.04.2019**
(21) Application number: 12759885.2
(22) Date of filing: 16.02.2012
(51) Int. Cl.: A61K 31/454

(54) **A POLYMORPH OF LENALIDOMIDE**
EIN POLYMORPH VON LENALIDOMID
FORME POLYMORPHE DE LÉNALIDOMIDE

(30) Priority: 23.03.2011 IN CH08992011
(43) Date of publication of application: 29.01.2014
(73) Proprietor: Hetero Research Foundation, Telangana (IN)
(72) Inventor: PARTHASARADHI REDDY, Bandi, Hyderabad 500018 Telangana (IN); RATHNAKAR REDDY, Kura, Hyderabad 500018 Telangana (IN); MURALIDHARA REDDY, Dasari, Hyderabad 500018 Telangana (IN); RAMAKRISHNA REDDY, Matta, Hyderabad 500018 Telangana (IN); VAMSI KRISHNA, Bandi, Hyderabad 500018 Telangana (IN)
(74) Representative: Best, Michael
(86) International application number: PCT/IN2012/000107
(87) International publication number: WO 2012/127493

(56) References cited:
- WO-A1-2010/056384
- WO-A1-2010/061209
- WO-A2-2005/023192
- WO-A2-2010/129636
- US-A1- 2011 021 567
- CAIRA M R: "CRYSTALLINE POLYMORPHISM OF ORGANIC COMPOUNDS", TOPICS IN CURRENT CHEMISTRY, SPRINGER, BERLIN, DE, vol. 198, 1 January 1998 (1998-01-01), pages 163-208, XP001156954, ISSN: 0340-1022, DOI: 10.1007/3-540-69178-2_5

## Description

This application claims the benefit of Indian Patent Application No. 899/CHE/2011, filed on March 23, 2011.

### Filed of the Invention

The present invention provides a novel crystalline Form of lenalidomide, process for its preparation and pharmaceutical compositions comprising it. The present invention also provides a novel N-methylpyrrolidone solvate of lenalidomide and process for its preparation.

### Background of the Invention

Lenalidomide is chemically, 3-(4-amino-1,3-dihydro-1-oxo-2*H*-isoindol-2-yl)-2,6-piperidinedione and has the structural formula:

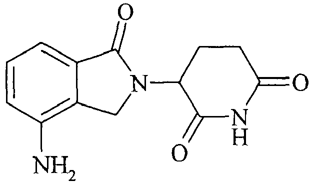

Lenalidomide, a thalidomide analogue, was initially intended for use as a treatment for multiple myeloma, for which thalidomide is an accepted therapeutic modality. Lenalidomide has also shown efficacy in the hematological disorders known as the myelodysplastic syndromes. Lenalidomide is currently marketed under the trade name REVLIMID® by Celgene.

Polymorphism is defined as "the ability of a substance to exist as two or more crystalline phases that have different arrangement and/or conformations of the molecules in the crystal Lattice. Thus, in the strict sense, polymorphs are different crystalline forms of the same pure substance in which the molecules have different arrangements and/or different configurations of the molecules". Different polymorphs may differ in their physical properties such as melting point, solubility, X-ray diffraction patterns, etc. Although those differences disappear once the compound is dissolved, they can appreciably influence pharmaceutically relevant properties of the solid form, such as handling properties, dissolution rate and stability. Such properties can significantly influence the processing, shelf life, and commercial acceptance of a polymorph. It is therefore important to investigate all solid forms of a drug, including all polymorphic forms, and to determine the stability, dissolution and flow properties of each polymorphic form. Polymorphic forms of a compound can be distinguished in the laboratory by analytical methods such as X-ray diffraction (XRD), Differential Scanning Calorimetry (DSC) and Infrared spectrometry (IR).

Solvent medium and mode of crystallization play very important role in obtaining a crystalline form over the other.

Lenalidomide can exist in different polymorphic forms, which may differ from each other in terms of stability, physical properties, spectral data and methods of preparation.

Lenalidomide and its process were disclosed in U.S. patent no. 5,635,517.

PCT publication no. WO 2005/023192 disclosed crystalline Form A, Form B, Form C, Form D, Form E, Form F, Form G and Form H of lenalidomide.

Amorphous lenalidomide was disclosed in PCT publication no. WO 2009/114601.

PCT publication no. WO 2010/056384 disclosed N,N-dimethylformamide solvate and dimethylsulfoxide solvate of lenalidomide.

Anhydrous crystalline Form of lenalidomide was disclosed in PCT publication no. WO 2010/061209. The publication also disclosed a process for preparing crystalline Form B of lenalidomide.

PCT publication no. WO 2010/129636 disclosed a crystalline Form I of lenalidomide.

We have found a novel crystalline Form of lenalidomide. The novel crystalline Form has been found to be stable over the time and reproducible and so, suitable for pharmaceutical preparations.

The lenalidomide N-methylpyrrolidone solvate as described herein may serve as intermediate for preparation of lenalidomide crystalline Form H1 or other polymorphs of lenalidomide.

Thus, object of the present invention is to provide a novel crystalline Form of lenalidomide, process for its preparation and pharmaceutical compositions comprising it.

### Summary of the Invention

The lenalidomide N-methylpyrrolidone solvate as described herein is characterized by peaks in the powder x-ray diffraction spectrum having 2θ angle positions at 8.5, 14.0, 14.5, 15.6, 16.1, 17.1, 17.6, 19.6, 21.6, 22.8 and 25.3 ± 0.2 degrees.

A process for the preparation of lenalidomide N-methylpyrrolidone solvate is described herein, which comprises:
a) suspending lenalidomide in N-methylpyrrolidone;
b) heating the suspension obtained in step (a) at above 50°C;
c) cooling the solution obtained in step (b) at below 15°C;
d) adding solvent to the solution at below 15°C; and
e) isolating lenalidomide N-methylpyrrolidone solvate.

The present invention provides a crystalline Form of lenalidomide designated as Form H1 characterized by peaks in the powder x-ray diffraction spectrum having 2θ angle positions at 10.6, 11.7, 14.9, 16.8, 18.2, 19.1, 22.5, 22.8, 23.6 and 28.2 ± 0.2 degrees.

In another aspect, the present invention provides a process for the preparation of crystalline Form H1 of lenalidomide, which comprises:
a) suspending any crystalline, solvated or amorphous Form of lenalidomide in N-methylpyrrolidone;
b) heating the suspension obtained in step (a) at above 50°C;
c) cooling the solution obtained in step (b) at about 15 to 40°C;
d) adding solvent to the solution at about 15 to 40°C;
e) separating the solid;
f) slurring the isolated solid obtained in step (e) with hydrocarbon solvent; and
g) isolating crystalline Form H1 of lenalidomide.

Yet in another aspect, the present invention provides a pharmaceutical composition comprising crystalline Form H1 of lenalidomide and pharmaceutically acceptable excipients.

### Brief Description of the Drawing

Figure 1 is X-ray powder diffraction spectrum of lenalidomide N-methylpyrrolidone solvate.
Figure 2 is X-ray powder diffraction spectrum of crystalline Form H1 of lenalidomide.

X-ray powder diffraction spectrum was measured on a bruker axs D8 advance X-ray powder diffractometer having a copper-Ka radiation. Approximately 1gm of sample was gently flattered on a sample holder and scanned from 2 to 50 degrees two-theta, at 0.02 degrees to theta per step and a step of 10.6 seconds. The sample was simply placed on the sample holder. The sample was rotated at 30 rpm at a voltage 40 KV and current 35 mA.

### Detailed Description of the Invention

The term "room temperature" refers to temperature at about 25 to 35°C.

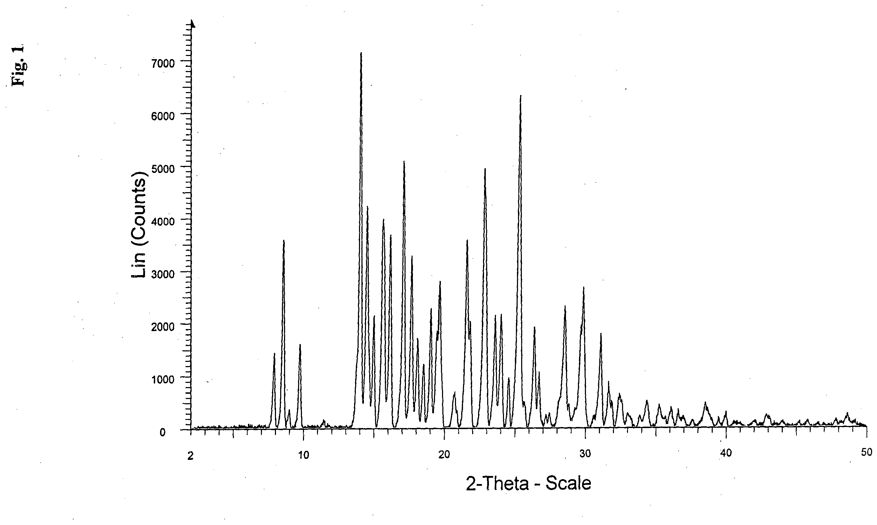

The lenalidomide N-methylpyrrolidone solvate as described herein is characterized by peaks in the powder x-ray diffraction spectrum having 2θ angle positions at 8.5, 14.0, 14.5, 15.6, 16.1, 17.1, 17.6, 19.6, 21.6, 22.8 and 25.3 ± 0.2 degrees. The powdered X-ray diffractogram (PXRD) of lenalidomide N-methylpyrrolidone solvate is shown in figure 1.

A process for the preparation of lenalidomide N-methylpyrrolidone solvate as described herein comprises:
a) suspending lenalidomide in N-methylpyrrolidone;
b) heating the suspension obtained in step (a) at above 50°C;
c) cooling the solution obtained in step (b) at below 15°C;
d) adding solvent to the solution at below 15°C; and
e) isolating lenalidomide N-methylpyrrolidone solvate.

The reaction may preferably be heated in step (b) at about 55 to 65°C.

The solution may preferably be cooled in step (c) at about 0 to 10°C.

The solvent used in step (d) may preferably be a solvent or mixture of solvents selected from methanol, ethanol, isopropyl alcohol, n-butanol, ethyl acetate, methyl acetate, isopropyl acetate, tert-butyl methyl acetate, ethyl formate, tetrahydrofuran, 1,4-dioxane, methyl tert-butyl ether, diisopropyl ether and diethyl ether. More preferably the solvents are ethanol, ethyl acetate and methyl tert-butyl ether.

The isolation of lenalidomide N-methylpyrrolidone solvate in step (e) may be carried out by the methods known such as filtration or centrifugation.

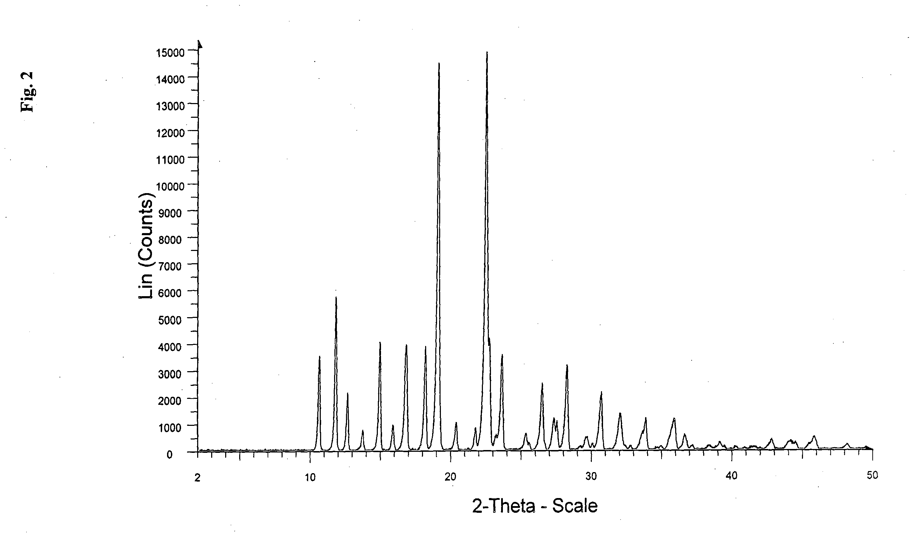

The present invention provides a crystalline Form of lenalidomide designated as Form H1 characterized by peaks in the powder x-ray diffraction spectrum having 2θ angle positions at 10.6, 11.7, 14.9, 16.8, 18.2, 19.1, 22.5, 22.8, 23.6 and 28.2 ± 0.2 degrees. The powdered X-ray diffractogram (PXRD) of crystalline Form H1 of lenalidomide is shown in figure 2.

According to another aspect of the present invention, there is provided a process for the preparation of crystalline Form H1 of lenalidomide, which comprises:
a) suspending any crystalline, solvated or amorphous Form of lenalidomide in N-methylpyrrolidone;
b) heating the suspension obtained in step (a) at above 50°C;
c) cooling the solution obtained in step (b) at 15 to 40°C;
d) adding solvent to the solution at 15 to 40°C;
e) separating the solid;
f) slurring the isolated solid obtained in step (e) with hydrocarbon solvent; and
g) isolating crystalline Form H1 of lenalidomide.

Lenalidomide used in step (a) may be any known crystalline, solvated or amorphous Forms.

The reaction may preferably be heated in step (b) at about 55 to 65°C.

The solution may preferably be cooled in step (c) at about 25 to 35°C.

The solvent used in step (d) may preferably be a solvent or mixture of solvents selected from methanol, ethanol, isopropyl alcohol, n-butanol, ethyl acetate, methyl acetate, isopropyl acetate, tert-butyl methyl acetate, ethyl formate, tetrahydrofuran, 1,4-dioxane, methyl tert-butyl ether, diisopropyl ether and diethyl ether. More preferably the solvents are ethanol, ethyl acetate and methyl tert-butyl ether.

The separation of the precipitated solid in step (e) may be carried by the methods known in the art such as filtration or centrifugation.

The hydrocarbon solvent used in step (f) may preferably be a solvent or mixture of solvents selected from cyclohexane, hexane, n-heptane, benzene, toluene and xylene, and more preferably the hydrocarbon solvent is toluene.

The temperature at which slurrying in step (f) is done is not critical and the slurrying may conveniently be carried out at about 50°C to 110°C.

Crystalline Form HI of lenalidomide may be isolated in step (g) by the methods known such as filtration or centrifugation.

According to another aspect of the present invention, there is provided a pharmaceutical composition comprising crystalline Form H1 of lenalidomide and pharmaceutically acceptable excipients, and optionally other therapeutic ingredients. The crystalline Form H1 may preferably be formulated into tablets, capsules, suspensions, dispersions, injectables or other pharmaceutical forms.

The invention will now be further described by the following examples, which are illustrative rather than limiting.

### Reference Examples

### Preparation of lenalidomide crystalline Form A

### Reference example 1:

Lenalidomide (100 gm) was dissolved in ethanol (1000 ml) and then heated to reflux to obtain a solution. The solution was maintained for 1 hour at reflux and then cooled to room temperature. The reaction mass was stirred for 1 hour at room temperature and filtered. The solid obtained was dried to give 85 gm of lenalidomide crystalline form A.

### Preparation of lenalidomide crystalline Form B

### Reference example 2:

Lenalidomide (100 gm) was dissolved in a mixture of ethanol (1500 ml) and water (1000 ml). The contents were heated to reflux and then treated with carbon. The reaction mass was filtered and then cooled to 0 to 5°C. The reaction mass was stirred for 1 hour at 0 to 5°C and filtered. The solid obtained was dried to give 84 gm of lenalidomide crystalline form B.

### Examples

### Preparation of lenalidomide N-methylpyrrolidone solvate

### Example 1 (Reference):

Lenalidomide (25 gm) was suspended in N-methylpyrrolidone (75 ml) and then heated to 60°C to obtain a solution. The solution was then cooled to 0 to 5°C and then added ethanol (125 ml). The reaction mass was stirred for 1 hour 30 minutes at 0 to 5°C and filtered. The solid obtained was dried to obtain 20 gm of lenalidomide N-methylpyrrolidone solvate.

### Example 2 (Reference):

Example 1 was repeated using ethyl acetate solvent instead of ethanol solvent to obtain lenalidomide N-methylpyrrolidone solvate.

### Example 3 (Reference):

Example 1 was repeated using methyl tert-butyl ether solvent instead of ethanol solvent to obtain lenalidomide N-methylpyrrolidone solvate.

### Preparation of lenalidomide crystalline Form H1

### Example 4:

Lenalidomide (25 gm) was suspended in N-methylpyrrolidone (75 ml) at room temperature and then heated to 60°C to obtain a solution. The solution was then cooled to room temperature and then added ethanol (125 ml). The reaction mass was stirred for 1 hour at room temperature, filtered and dried to obtain a solid. To the solid was added toluene (180 ml) and then heated to 90 to 95°C. The reaction mass was maintained for 1 hour at 90 to 95°C and then cooled to room temperature. The reaction mass was stirred for 1 hour at room temperature and filtered. The solid obtained was dried under vacuum at 95 to 100°C for 36 hours to obtain 17 gm of lenalidomide crystalline Form H1.

### Example 5:

Example 4 was repeated using ethyl acetate solvent instead of ethanol solvent to obtain lenalidomide crystalline Form H1.

### Example 6:

Example 4 was repeated using methyl tert-butyl ether solvent instead of ethanol solvent to obtain lenalidomide crystalline Form H1.

### Example 7:

Lenalidomide N-methylpyrrolidone solvate (100 gm) as obtained in example 1 was suspended in N-methylpyrrolidone (300 ml) at room temperature. The contents were heated to 60°C to obtain a solution. The solution was then cooled to room temperature and then added ethyl acetate (500 ml). The reaction mass was stirred for 1 hour at room temperature. The separated solid was filtered and dried to obtain a solid. To the solid was added toluene (720 ml) and then heated to 90 to 95°C. The reaction mass was maintained for 1 hour at 90 to 95°C and then cooled to room temperature. The contents were stirred for 1 hour at room temperature, filtered and then dried under vacuum at 95 to 100°C for 40 hours to obtain 67 gm of lenalidomide crystalline Form H1.

### Example 8:

Example 7 was repeated using lenalidomide N,N-dimethylformamide solvate instead of lenalidomide N-methylpyrrolidone solvate to obtain lenalidomide crystalline Form H1.

### Example 9:

Example 7 was repeated using lenalidomide N-methylsulfoxide solvate instead of lenalidomide N-methylpyrrolidone solvate to obtain lenalidomide crystalline Form H1.

### Example 10:

Example 7 was repeated using ethanol solvent instead of ethyl acetate solvent to obtain lenalidomide crystalline Form H1.

### Example 11:

Example 7 was repeated using methyl tert-butyl ether solvent instead of ethyl acetate solvent to obtain lenalidomide crystalline Form H1.

### Example 12:

Lenalidomide crystalline Form B (15 gm) as obtained in reference example 2 was suspended in N-methylpyrrolidone (45 ml) at room temperature and then heated to 60°C to obtain a solution. The solution was then cooled to room temperature and then added methyl tert-butyl ether (105 ml). The reaction mass was stirred for 1 hour at room temperature, filtered and dried to obtain a solid. To the solid was added toluene (100 ml) and then heated to 90 to 95°C. The reaction mass was maintained for 1 hour at 90 to 95°C and then cooled to room temperature. The reaction mass was stirred for 1 hour at room temperature and filtered. The solid obtained was dried under vacuum at 95 to 100°C for 36 hours to obtain 10 gm of lenalidomide crystalline Form H1.

### Example 13:

Example 12 was repeated using lenalidomide crystalline Form A as obtained in reference example 1 instead of lenalidomide crystalline Form B to obtain lenalidomide crystalline Form H1.

### Example 14:

Example 12 was repeated using ethanol solvent instead of methyl tert-butyl ether solvent to obtain lenalidomide crystalline Form H1.

### Example 15:

Example 12 was repeated using ethyl acetate solvent instead of methyl tert-butyl ether solvent to obtain lenalidomide crystalline Form H1.

## Claims

1. A crystalline Form HI of lenalidomide which is **characterized by** peaks in the powder x-ray diffraction spectrum having 2θ angle positions at about 10.6, 11.7, 14.9, 16.8, 18.2, 19.1, 22.5, 22.8, 23.6 and 28.2 ± 0.2 degrees.

2. A process for the preparation of crystalline Form H1 of lenalidomide as claimed in claim 1, which comprises:
a. suspending any crystalline, solvated or amorphous Form of lenalidomide in N-methylpyrrolidone;
b. heating the suspension obtained in step (a) at above 50°C;
c. cooling the solution obtained in step (b) at 15 to 40°C;
d. adding solvent to the solution at 15 to 40°C;
e. separating the solid;
f. slurring the isolated solid obtained in step (e) with hydrocarbon solvent; and
g. isolating crystalline Form H1 of lenalidomide.

3. The process as claimed in claim 2, wherein in step (b) the reaction is heated to 55-65°C.

4. The process as claimed in claim 2, wherein the solvent used in step (d) is a solvent or mixture of solvents selected from methanol, ethanol, isopropyl alcohol, n-butanol, ethyl acetate, methyl acetate, isopropyl acetate, tert-butyl methyl acetate, ethyl formate, tetrahydrofuran, 1,4-dioxane, methyl tert-butyl ether, diisopropyl ether and diethyl ether.

5. The process as claimed in claim 4, wherein the solvent is selected from ethanol, ethyl acetate and methyl tert-butyl ether.

6. The process as claimed in claim 2, wherein the hydrocarbon solvent used in step (f) is a solvent or mixture of solvents selected from cyclohexane, hexane, n-heptane, benzene, toluene and xylene.

7. The process as claimed in claim 6, wherein the hydrocarbon solvent is toluene.

8. A pharmaceutical composition in tablet or capsule form that comprises crystalline lenalidomide Form H1 as claimed in claim 1 and pharmaceutically acceptable excipients, and optionally other therapeutic ingredients.

## Patentansprüche

1. Kristalline Form H1 von Lenalidomid, die durch Peaks im Pulver-Röntgenbeugungsspektrum mit 2θ-Winkelpositionen bei 10,6, 11,7, 14,9, 16,8, 18,2, 19,1, 22,5, 22,8, 23,6 und 28,2 ± 0,2 Grad gekennzeichnet ist.

2. Verfahren zur Herstellung der kristallinen Form H1 von Lenalidomid nach Anspruch 1, das umfasst:
a. Suspendieren einer kristallinen, solvatisierten oder amorphen Form von Lenalidomid in N-Methylpyrrolidon;
b. Erwärmen der in Schritt (a) erhaltenen Suspension auf über 50°C;
c. Abkühlen der in Schritt (b) erhaltenen Lösung auf 15 bis 40°C;
d. Zugeben eines Lösungsmittels zu der Lösung bei 15 bis 40°C;
e. Abtrennen des Feststoffes;
f. Aufschlämmen des in Schritt (e) erhaltenen isolierten Feststoffes mit einem Kohlenwasserstofflösungsmittel und
g. Isolieren der kristallinen Form H1 von Lenalidomid.

3. Verfahren nach Anspruch 2, wobei in Schritt (b) die Reaktion auf 55 - 65°C erwärmt wird.

4. Verfahren nach Anspruch 2, wobei das in Schritt (d) verwendete Lösungsmittel ein Lösungsmittel oder ein Gemisch aus Lösungsmitteln ist, ausgewählt aus Methanol, Ethanol, Isopropylalkohol, n-Butanol, Ethylacetat, Methylacetat, Isopropylacetat, tert-Butylmethylacetat, Ethylformiat, Tetrahydrofuran, 1,4-Dioxan, Methyl-tert-butylether, Diisopropylether und Diethylether.

5. Verfahren nach Anspruch 4, wobei das Lösungsmittel ausgewählt ist aus Ethanol, Ethylacetat und Methyl-tert-butylether.

6. Verfahren nach Anspruch 2, wobei das in Schritt (f) verwendete Kohlenwasserstofflösungsmittel ein Lösungsmittel oder ein Gemisch aus Lösungsmitteln ist, ausgewählt aus Cyclohexan, Hexan, n-Heptan, Benzol, Toluol und Xylol.

7. Verfahren nach Anspruch 6, wobei das Kohlenwasserstofflösungsmittel Toluol ist.

8. Pharmazeutische Zusammensetzung in Tabletten- oder Kapselform, die die kristalline Lenalidomidform H1 nach Anspruch 1 und pharmazeutisch akzeptable Hilfsstoffe und gegebenenfalls andere therapeutische Inhaltsstoffe umfasst.

## Revendications

1. Forme cristalline H1 de lénalidomide qui est **caractérisée par** des pics du spectre de diffractométrie de rayons X sur poudres présentant des positions d'angle 2θ à 10,6, 11,7, 14,9, 16,8, 18,2, 19,1, 22,5, 22,8, 23,6 et 28,2 ± 0,2 degrés.

2. Procédé de préparation de la forme cristalline H1 de lénalidomide selon la revendication 1, qui comprend les étapes consistant à :
a. mettre en suspension toute forme cristalline, solvatée ou amorphe de lénalidomide dans de la N-méthylpyrrolidone ;
b. chauffer la suspension obtenue à l'étape (a) au-dessus de 50 °C ;
c. refroidir la solution obtenue à l'étape (b) entre 15 et 40 °C ;
d. ajouter du solvant à la solution entre 15 et 40 °C ;
e. séparer le solide ;
f. étaler le solide isolé obtenu à l'étape (e) avec un solvant hydrocarboné ; et
g. isoler la forme cristalline H1 de lénalidomide.

3. Procédé selon la revendication 2, dans lequel dans l'étape (b), la réaction est chauffée à 55- 65 °C.

4. Procédé selon la revendication 2, dans lequel le solvant utilisé dans l'étape (d) est un solvant ou un mélange de solvants choisis parmi le méthanol, l'éthanol, l'alcool isopropylique, le n-butanol, l'acétate d'éthyle, l'acétate de méthyle, l'acétate d'isopropyle, l'acétate de méthyle de tert-butyle, le formiate d'éthyle, le tétrahydrofuranne, le 1,4-dioxane, l'éther de méthyle de tert-butyle, l'éther diisopropylique et l'éther diéthylique.

5. Procédé selon la revendication 4, dans lequel le solvant est choisi parmi l'éthanol, l'acétate d'éthyle et l'éther de méthyle de tert-butyle.

6. Procédé selon la revendication 2, dans lequel le solvant hydrocarboné utilisé dans l'étape (f) est un solvant ou un mélange de solvants choisis parmi le cyclohexane, l'hexane, le n-heptane, le benzène, le toluène et le xylène.

7. Procédé selon la revendication 6, dans lequel le solvant hydrocarboné est le toluène.

8. Composition pharmaceutique sous forme de comprimé ou de gélule, qui comprend la forme cristalline H1 de lénalidomide selon la revendication 1, et des excipients pharmaceutiquement acceptables et éventuellement d'autres ingrédients thérapeutiques.
